# EUROPEAN PATENT SPECIFICATION

(11) **EP 3 403 600 B1**
(45) Date of publication and mention of the grant of the patent: **13.05.2020**
(21) Application number: 18177577.6
(22) Date of filing: 16.08.2017
(51) Int. Cl.: A61B 17/15

(54) **SURGICAL DEVICE FOR OSTEOTOMY**
CHIRURGISCHE VORRICHTUNG ZUR OSTEOTOMIE
DISPOSITIF CHIRURGICAL POUR OSTÉOTOMIE

(30) Priority: 19.08.2016 TW 105126498; 10.08.2017 TW 106127173
(43) Date of publication of application: 21.11.2018
(62) Divisional of application: 17186473.9
(73) Proprietor: A Plus Biotechnology Company Limited, New Taipei City 235 (TW)
(72) Inventor: WU, Kai-Hsing, Taipei City 105 Songshan Dist. (TW); LO, Hsiang Wei, New Taipei City 237 Sanxia Dist. (TW); LUO, Chu An, Huwei Township Yunlin County (TW); CHEN, Chun-Ming, New Taipei City 239 Yingge Dist. (TW); YU, Ping Sheng, Taipei City 112 Beitou Dist. (TW)
(74) Representative: Zaboliene, Reda

(56) References cited:
- WO-A1-2015/162437
- CN-U- 205 359 552
- US-A1- 2008 262 500
- US-B2- 8 632 547

## Description

### TECHNICAL FIELD

The present invention relates to a surgical device for osteotomy. More specifically, it is related to a surgical device of osteotomies and custom-made for the specific patient.

### BACKGROUND

The knee is one of the most weight-bearing joints in the body. If the knee had sports injuries or into the middle age, it is likely to cause articular cartilage wear and resulting in a symptom of knee pain. The daily activity is limited. This is the cause of the degenerative joint disease.

Common treatment is artificial joint arthroplasty that involves large bone resection for the fixation of the metal and polymer implants. Due to the wear of the implant, the maximum duration of the artificial joint replacement is only twenty years, and may cause complications like infection, osteolysis and osteoporosis, which will need revision surgery. Furthermore, some patients with early degenerative joint disease, in fact, only wear the medial articular surface. It is not necessary to remove the entire joint for arthroplasty. For example, high tibial osteotomy is commonly used for patients with medial degenerative knee osteoarthritis.

High tibial osteotomy is a surgery to realign the biomechanical axis by cutting and opening at the medial proximal tibia, sometimes the opening gap needs to be filled with bone grafts. Then the osteomized tibia is fixed by a bone plate. This surgical procedure avoids bone resection and preserves the knee joint. It is a better choice for the patient with medial knee pain.

However, high tibial osteotomy requires cutting and opening at the proximal tibia. The cutting position, direction, depth, and surgical opening height are important and should be carefully planned, but are difficult to perform precisely. Currently, the above-mentioned parameters can only checked intraoperatively by X-ray and the surgeon's experience. The amount of the correction of biomechanical axis and above-mentioned parameters are all different for each patient, thus the design of surgical instruments must be considered as well. An U.S. patent application 2008/0262500A1 discloses a cutting guide for performing a bone osteotomy procedure. The cutting guide includes a first arm having a first cutting guide surface formed therein, a second arm having a second cutting guide surface formed therein pivotably connected to the first arm and a distractor operatively connected to the first arm. The cutting guide is adapted to be affixed to the bone such that the first cutting guide surface is open to the second cutting guide surface. The first arm and second arm are rotatable with respect to each other such that manipulation of the distractor creates a force between the first arm and the second arm causing rotation of the first arm and second arm relative to each other. A method for using the cutting guide in a bone osteotomy procedure is also disclosed. An international patent application 2015/162437A1 discloses a combined bone cutting guide and spreader device comprising first and second arms and a hinge connecting first ends of said first and second arms together, in which second ends of said first and second arms each comprise a bone attachment mechanism, in which said first and second arms are moveable about said hinge between a guide position in which they are arranged parallel with one another and define a bone cutting guide slot therebetween, and such that said bone attachment mechanisms are adjacent to one another, and a spread position in which they are arranged at an angle to one another and said bone attachment mechanisms are spaced apart.

### SUMMARY OF THE INVENTION

In view of the foregoing problems, the present invention provides a surgical device for osteotomy as defined in claim 1. Preferred embodiments of the invention are defined in the dependent claims. It provides a guide for saw blade to perform high tibial osteotomy, but not limited to, the surgical device for osteotomy can be used for other bones, such as: femur, fibula, humerus, ulna, radius, clavicle, scapula and so on. The tibia is described in the preferred embodiment of the present invention. The exterior design has features that can assist the clinician to determine the cutting position, direction and depth, the opening height precisely. Compared with conventional free-hand surgery, the present invention is more precise, time saving, easy learning, and with low exposure of radiation. Each device is custom-made for the patient.

Compared with the conventional technique, the surgical device for osteotomy is manufactured by three-dimensional printing (3D printing) according to the patient's skeletal image data, the optimal cutting position is evaluated preoperatively. The device can fit well with the patient's bone. The surgeon can perform the first cutting position under the guide groove specified by the device. The guide groove allows the surgeon to perform the osteotomy accurately. It also provides a reference for calculating the cutting angle and depth. The side guide edge provides the reference for the second cut. Not only improving the surgical accuracy and efficiency, but the present invention also standardizes the whole surgical procedure for surgeons.

### BRIEF DESCRIPTION OF THE DRAWINGS

FIG. 1A illustrates a front view of an osteotomy device, which does not form part of the claimed invention.
FIG. 1B illustrates a side view of an osteotomy device, which does not form part of the claimed invention.
FIG. 2 illustrates the position at which the tibia is to be cut.
FIG. 3A illustrates a back view of an osteotomy device, which does not form part of the claimed invention, when implemented in a tibia.
FIG. 3B illustrates a side view of an osteotomy device, which does not form part of the claimed invention, when implemented in a tibia.
FIG. 4A illustrates a front view of an osteotomy device, which does not form part of the claimed invention, before surgical process.
FIG. 4B illustrates a side view of an osteotomy device, which does not form part of the claimed invention, before surgical process.
FIG. 5 illustrates an embodiment of the present invention.
FIG. 6 illustrates another embodiment of the present invention when implemented.
FIG. 7 illustrates a front view of another osteotomy device, which does not form part of the claimed invention.
FIG. 8A illustrates a side view of the other osteotomy device, which does not form part of the claimed invention.
FIG. 8B illustrates the position at which the femur is to be cut when an osteotomy is practiced.
FIG. 9A illustrates a front view of the other osteotomy device, which does not form part of the claimed invention.
FIG. 9B illustrates a side view of the other osteotomy device, which does not form part of the claimed invention.

### DETAILED DESCRIPTION

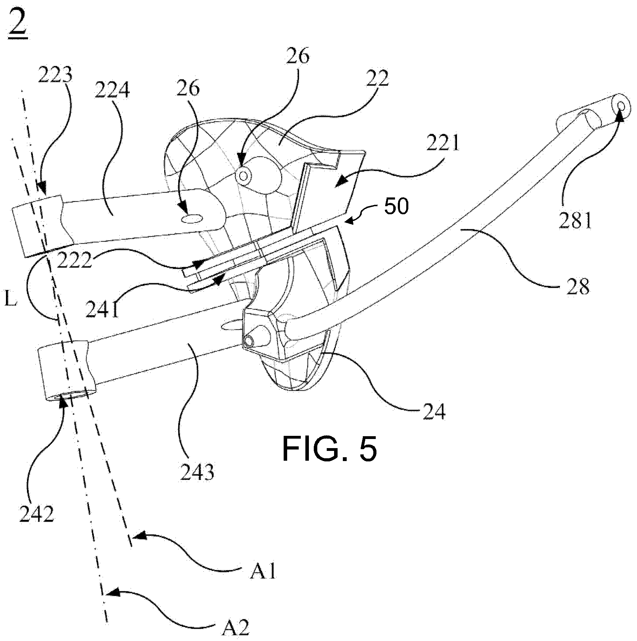

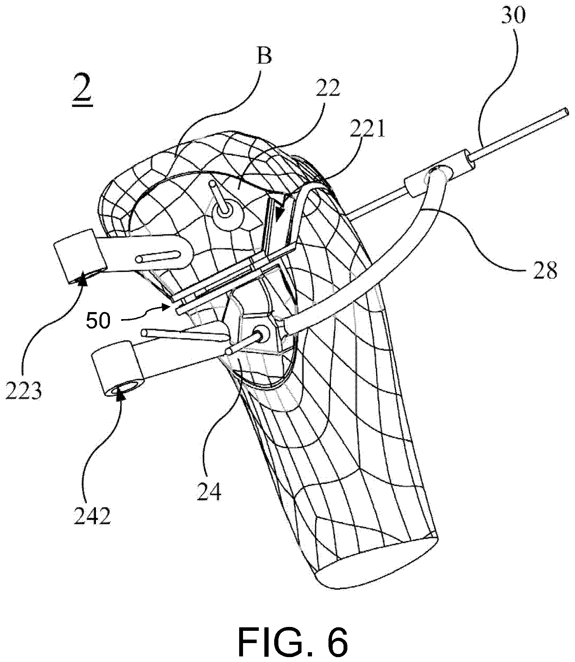

Some preferred embodiments of the present invention will now be described in greater detail. Only the osteotomy devices shown in figures 5 and 6 are according to the claimed invention. However, it should be recognized that the preferred embodiments of the present invention are provided for illustration rather than limiting the present invention.

In addition, the present invention can be practiced in a wide range of other embodiments besides those explicitly described, and the scope of the present invention is not expressly limited except as specified in the accompanying claims. The layout of components may be more complicated in practice.

Furthermore, the greater number or lesser number mentioned in the present specification is included in the number itself. It is to be understood that this specification discloses certain methods and processes for carrying out the functions, there are a variety of structures related to the disclosed structures that perform the same function. The above-mentioned structures generally achieve the same result.

Hereinafter, preferred embodiments of the present invention will be described in detail to fully illustrate the features, and advantages of the present invention.

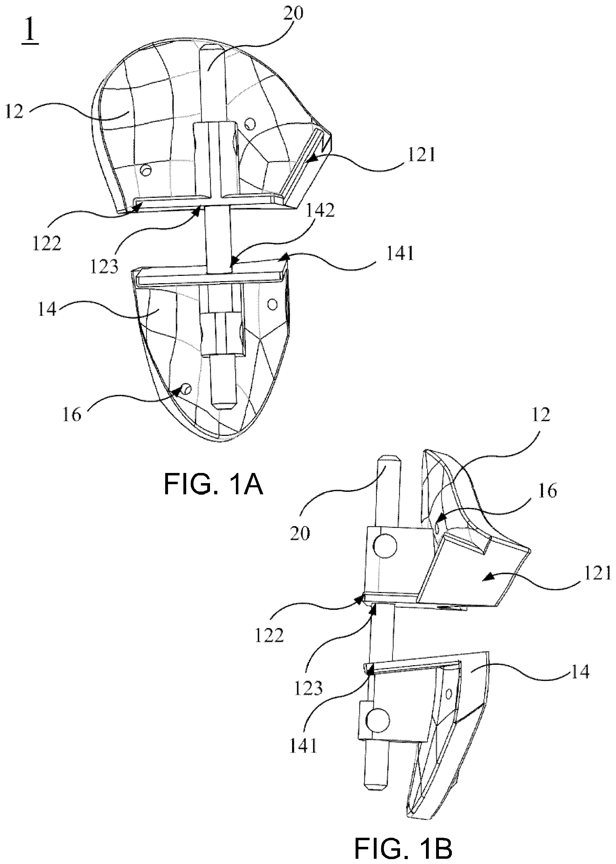

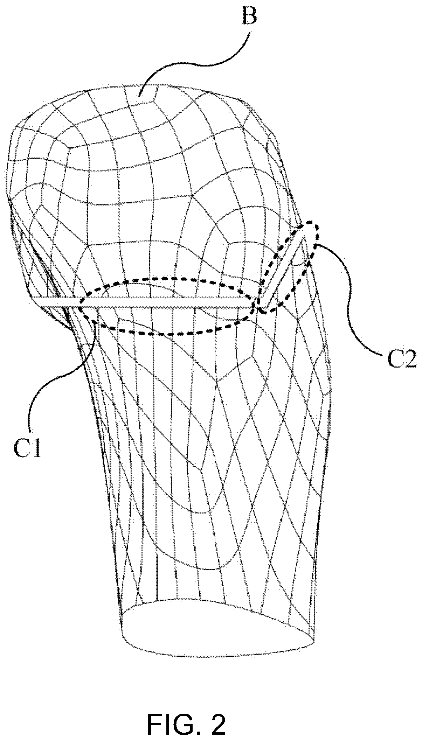

Please refer to FIG. 1A, FIG. 1B and FIG. 2. FIG. 1A illustrates a front view of an osteotomy device, which does not form part of the claimed invention. FIG. 1B illustrates a side view of an osteotomy device, which does not form part of the claimed invention, when implemented. FIG. 2 illustrates the position at which the tibia is to be cut.

The surgical device for osteotomy 1 can be used in various osteotomy, correction or reduction surgery. The surgical device for osteotomy 1 can be used for various bones of the human body, such as: tibia B, femur D, fibula, humerus, ulna, radius, clavicle, scapula, etc., but not limited to. It provides a guide for saw blade to perform high tibial osteotomy of a tibia B. The first cutting position C1 and a second cutting position C2 on tibia B are defined by the surgical device for osteotomy 1. The surgical device for osteotomy 1 comprises: a first body component 12, a second body component 14, and a plurality of fixing slots 16. Wherein the first body component 12 is disposed above the first cutting position C1 and has a side guide edge 121 and an upper guide edge 122. The second body component 14 is disposed below the first cutting position C1 or below the upper guide edge 122 and has a lower guide edge 141. The lower guide edge 141 is disposed below the upper guide edge 122. The surfaces of the first body component 12 and the second body component 14 have the plurality of fixing slots 16. The surgical device for osteotomy 1 is fixed on the surface of the tibia B by inserting a plurality of fixing pins into the plurality of fixing slots 16.

Wherein the upper guide edge 122 and the lower guide edge 141 extend outwardly from the first body component 12 and the second body component 14, respectively. A guide groove 50 is formed between the upper guide edge 122 and the lower guide edge 141 for guiding the saw blade to the first cutting position C1. The side guide edge 121 is used to guide the saw blade to the second cutting position C2. The upper guide edge 122, the lower guide edge 141 and the side guide edge 121 are used to form the cutting track. The saw blade cuts the tibia B according to the first cutting position C1 and the second cutting position C2, thereby operating high tibial osteotomy.

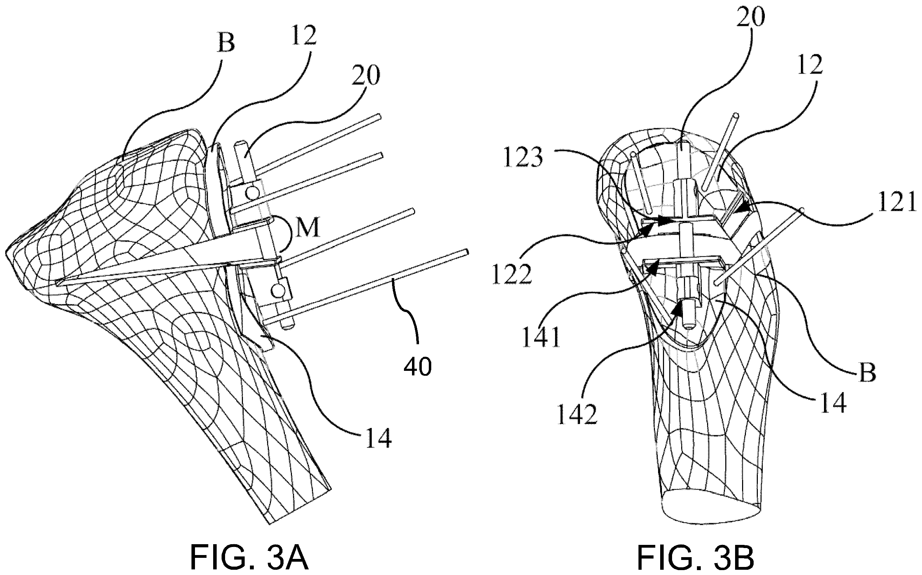

Please refer to FIG. 3A and FIG. 3B. FIG. 3A illustrates a back view of an osteotomy device, which does not form part of the claimed invention, when implemented in a tibia. FIG. 3B illustrates a side view of an osteotomy device, which does not form part of the claimed invention, when implemented in a tibia. The first body component 12 further comprises a first correcting perforation 123. The first correcting perforation 123 is provided on the upper guide edge 122. The second body component 14 further comprises a second correcting perforation 142. The second correcting perforation 142 is provided on the lower guide edge 141. The first correcting perforation 123 and the second correcting perforation 142 are designed to confirm the correction angle which is opened intraoperatively in high tibial osteotomy. For this reason, there is a regulative angle L between the first hole axis A1 of the first correcting perforation 123 and the second hole axis A2 of the second correcting perforation 142 (the hole axes A1, A2 and the regulative angle are plotted in FIG. 5). In high tibial osteotomy, a gap of the osteotomy has a preoperative planning correction angle M. When the tibia B is opened by the first body component 12 and the second body component 14 with the correction angle M, the first hole axis A1 of the first correcting perforation 123 and the second hole axis A2 of the second correcting perforation 142 can coincide. An aligning bar 20 passes through the first correcting perforation 123 and the second correcting perforation 142 to ensure the opening has achieved the correction angle. Firstly, the size of the aforementioned correction angle is based on the correction angle M that the tibia B needs to open in high tibial osteotomy. Secondly, the angle between the axis of the first correcting perforation and the axis of the second correcting perforation is determined according to the desired correction angle M and it is made. The aligning bar 20 can be inserted between the first correcting perforation 123 and the second correcting perforation 142 only when the tibia B is opened to the angle of preoperative planning by the first body component 12 and the second body component 14.

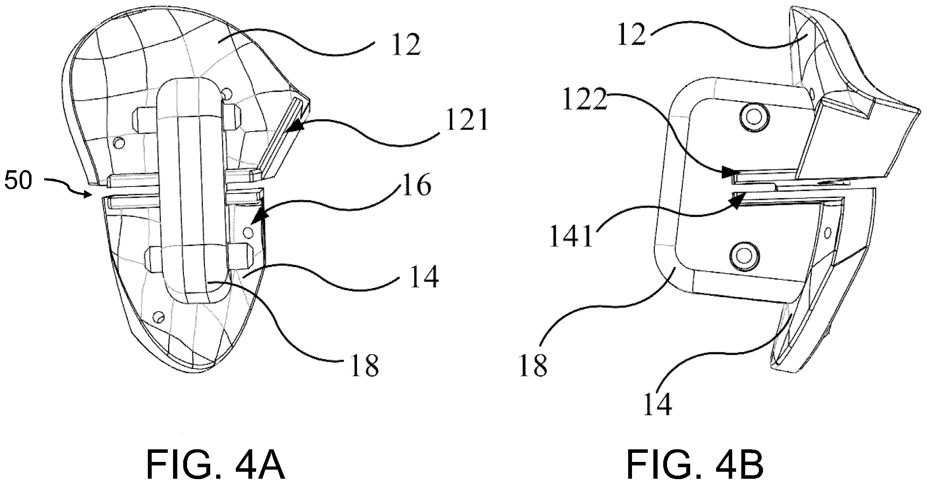

Please refer to FIG. 4A and FIG. 4B. FIG. 4A illustrates a front view of an osteotomy device, which does not form part of the claimed invention, before surgical process. FIG. 4B illustrates a side view of an osteotomy device, which does not form part of the claimed invention, before surgical process. The present invention, the first body component 12 and the second body component 14 are made by 3D printing. Since the first body component 12 and the second body component 14 need to be connected with each other in the surgery before the sawing procedure. So, the surgical device for osteotomy 1 further comprises a connecting component 18. Before the sawing procedure, the first body component 12 and the second body component 14 are connected by component 18 to ensure the sufficient area for attaching the bone surface. The first body component 12 and the second body component 14 are fixed to the patient's tibia before the sawing procedure of high tibial osteotomy, and then the connecting component 18 is removed. However, the first body component 12 and the second body component 14 may be manufactured in an integrally formed manner. At this time, there is a connecting part between the upper guide edge 122 and the lower guide edge 141, then the connecting component 18 is not necessary. When the osteotomy is performed, the surgeon can directly cut the connecting part with the bone saw.

Please refer to FIG. 5 and FIG. 6. FIG. 5 illustrates an embodiment of the present invention. FIG. 6 illustrates an embodiment of the present invention when implemented.

The present invention provides a surgical device for osteotomy 2 which can be used in various osteotomy, correction or reduction surgery. The surgical device for osteotomy 2 can be used for various bones of the human body, such as: tibia B, femur D, fibula, humerus, ulna, radius, clavicle, scapula, etc., but not limited to. In the present embodiment, it provides a guide for saw blade to perform high tibial osteotomy of a tibia B. The first cutting position C1 and a second cutting position C2 on tibia B are defined by the surgical device for osteotomy 2. The surgical device for osteotomy 2 comprises: a first body component 22, a second body component 24, a plurality of fixing slots 26 and a hinge pin guide component 28. In this embodiment, the first body component 22 and the second body component 24 are connected by a connecting part. When the osteotomy is performed, the surgeon can directly cut the connecting part with the bone saw, but not limited to. The first body component 22 and the second body component 24 can be directly used without using the connecting part. In addition, the first body component 22 and the second body component 24 may be connected in other forms.

Wherein the first body component 22 is disposed above the first cutting position C1 and has a side guide edge 221, an upper guide edge 222 and a first correcting perforation 223. The first correcting perforation 223 is connected to the first body component 22 by a first bar 224. The second body component 24 is disposed below the first cutting position C1 or below the upper guide edge 222 and has a lower guide edge 241 and a second correcting perforation 242. The lower guide edge 241 is disposed below the upper guide edge 222. A guide groove 50 is formed between the upper guide edge 222 and the lower guide edge 241. The second correcting perforation 242 is connected to the second body component 24 by a second bar 243. The surfaces of the first body component 22 and the second body component 24 have the plurality of fixing slots 26. The surgical device for osteotomy 2 is fixed on the surface of the tibia B by inserting a plurality of fixing pins 40 into the plurality of fixing slots 26. A hinge pin guide component 28 is disposed on the surface of the second body component 24, it comprises a third correcting perforation 281 extending to the rear of the second cutting position C2 or the rear of the guide groove 50. The third correcting perforation 281 fits with the preoperative hinge axis 30 for high tibial osteotomy as a rotation axis when the tibia B is opened. By inserting the pin as the hinge axis 30 , the position and direction of the hinge can be guaranteed as the preoperative planning intraoperatively. When the saw blade has reached the pre-planned cutting depth, it will be blocked by the hinge pin on the hinge axis 30 to ensure the sawing depth and improve the surgical accuracy. In addition, pre-drilled hole can distribute the stress and avoid the fracture of lateral cortex of tibia B while opening the gap. The hinge pin guide component 28 and the second body component 24 are integrally formed or combined.

Wherein the upper guide edge 222 and the lower guide edge 241 extend outwardly from the first body component 22 and the second body component 24, respectively. A guide groove 50 is formed between the upper guide edge 222 and the lower guide edge 241 for guiding the saw blade to the first cutting position C1. The side guide edge 221 is used to guide the saw blade to the second cutting position C2. The upper guide edge 222, the lower guide edge 241 and the side guide edge 221 are used to form the cutting track. The saw blade cuts the tibia B according to the first cutting position C1 and the second cutting position C2, thereby operating high tibial osteotomy.

In the present invention, the first correcting perforation 223 and the second correcting perforation 242 are designed to confirm the correction angle which is opened intraoperatively in high tibial osteotomy. For this reason, there is a regulative angle L between the first hole axis A1 of the first correcting perforation 223 and the second hole axis A2 of the second correcting perforation 242. When the tibia B is opened by the first body component 22 and the second body component 24 with the correction angle that is the same as the preoperative planning, the first hole axis A1 of the first correcting perforation 223 and the second hole axis A2 of the second correcting perforation 242 can coincide. An aligning bar 20 passes through the first correcting perforation 223 and the second correcting perforation 242 to ensure the opening has achieved the correction angle. Firstly, the size of the aforementioned regulative angle L is based on the correction angle that the tibia B needs to open in high tibial osteotomy. Secondly, the size of the regulative angle L is determined according to the desired correction angle and it is made. The aligning bar 20 can be inserted between the first correcting perforation 223 and the second correcting perforation 242 only when the tibia B is opened to the angle of preoperative planning by the first body component 22 and the second body component 24.

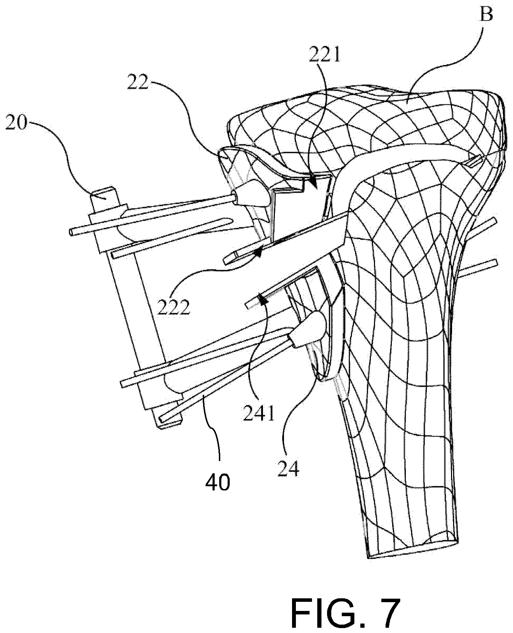

Please refer to FIG. 2, FIG. 3A, FIG. 3B and FIG. 7. Hereby explain how to perform high tibial osteotomy. In FIG. 2, the surgical devices for osteotomy 1, 2 attached to the tibial position are located in the calf of human body and close to the knee on the medial side of the proximal end. However, the scope of its attachment is not limited to this, it can also be attached to other positions of the other bones. In other words, the picture shows the positive side of FIG. 2 is pointing to the inside of human body. The left side of FIG. 2 points to the back of human body. FIG. 3A and FIG. 3B correspond to the surgical device for osteotomy 1. FIG. 7 illustrates a front view of another osteotomy device, which does not form part of the claimed invention, when implemented and corresponds to the surgical device for osteotomy 2.

The surgical devices for osteotomy 1, 2 are based on the tibial image information provided by patients with degenerative joint disease before surgery. The model of tibia B was established using computer software. The position of the first cutting position C1 and the second cutting position C2 is determined according to the model of tibia B. The overall structure of the surgical devices for osteotomy 1, 2 are custom-made. Wherein the surgical devices for osteotomy 1, 2 are designed to be able to fit well with the surface of the patient's tibia B, and then the three-dimensional printing technique is used to construct an integrally formed or combined solid instrument.

When the surgical devices for osteotomy 1, 2 are arranged on the surface of patient's tibia, the surgeon inserts the saw blade and starts cutting according to the first cutting position C1 guided by the upper guide edge 122, 222 and the lower guide edge 141, 241 of the surgical devices for osteotomy 1, 2. The surgeon can use the upper guide edges 122, 222 and the lower guide edges 141, 241 as a reference for the calculation of the cutting depth. In another way, make a mark on the saw blade, the surgeons can use the naked eye to confirm whether the sawing depth of the saw blade has reached the preset depth.

The saw blade keeps cutting along the upper guide edge 122, 222 and the lower guide edge 141, 241 to the inside of the human body until the predetermined depth is achieved. Cut off part of the tibia B. Along the second cutting position C2 guided by the side guide edges 121, 221 to create an ascending cut. After the cutting, the first cutting position C1 of the tibia B is opened to the preoperative planning correction angle M in the case where the surgical devices for osteotomy 1, 2 are fixed to the tibia B. The aligning bar 20 is then inserted through the first correcting perforations 123, 223 and the second correcting perforations 142, 242. After confirming the correction angle of the osteotomy of the tibia B, the gap can be fixed with the implant.

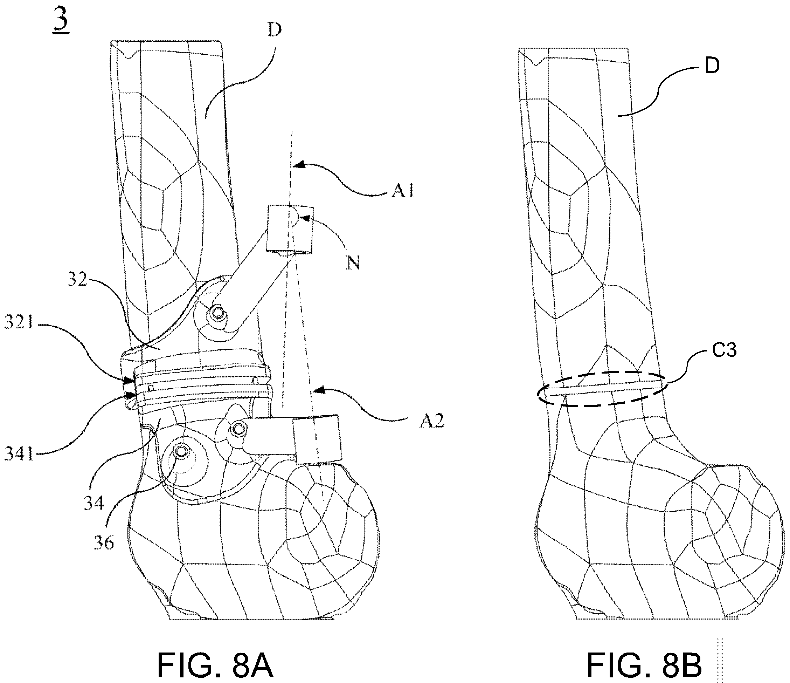

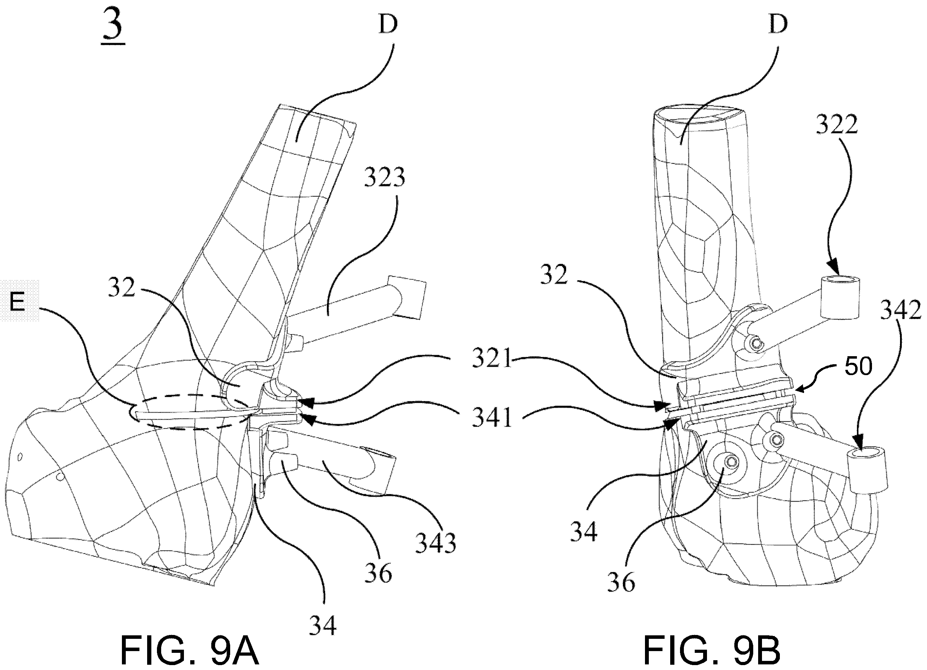

Please refer to FIG. 8A, FIG. 8B, FIG. 9A and FIG. 9B. FIG. 8A illustrates a side view of the other osteotomy device, which does not form part of the claimed invention, when implemented. FIG. 8B illustrates the position at which the femur D is to be cut. FIG. 9A illustrates a front view of the other osteotomy device, which does not form part of the claimed invention, when implemented. FIG. 9B illustrates a side view of the other osteotomy device, which does not form part of the claimed invention, when implemented.

The present description further provides a surgical device for osteotomy 3 which can be used in various osteotomy, correction or reduction surgery. The surgical device for osteotomy 3 can be used for various bones of the human body, such as: tibia B, femur D, fibula, humerus, ulna, radius, clavicle, scapula, etc., but not limited to. In the present example, it provides a guide for saw blade to perform the distal femoral osteotomy (DFO) of a femur D. The surgical device for osteotomy 3 comprises: a first body component 32, a second body component 34 and a plurality of fixing slots 36. In this example, the first body component 32 and the second body component 34 are connected by a connecting part. When the osteotomy is performed, the surgeon can directly cut the connecting part with the bone saw, but not limited to. The first body component 32 and the second body component 34 can be directly used without using the connecting part. In addition, the first body component 32 and the second body component 34 may be connected in other forms. FIG. 8A to FIG. 9B shows the femur is the body's left thigh (femur) near the knee. However, the scope of application is not limited thereto, it can also be applied to other positions of other bones.

The aforementioned femur D is defined as a third cutting position C3 by the surgical device for osteotomy 3. Wherein the first body component 32 is disposed above the third cutting position C3 and has an upper guide edge 321 and a first correcting perforation 322, the first correcting perforation 322 is connected to the first body component 32 by a first bar 323. The second body component 34 is disposed below the third cutting position C3 or below the upper guide edge 321 and has a lower guide edge 341 and a second correcting perforation 342. The lower guide edge 341 is disposed below the upper guide edge 321. The second correcting perforation 342 is connected to the second body component 34 by a second bar 343. The surfaces of the first body component 32 and the second body component 34 have the plurality of fixing slots 36, and the surgical device for osteotomy 3 is fixed on the surface of the femur D by inserting a plurality of fixing pins 40 into the plurality of fixing slots 36.

Wherein the upper guide edge 321 and the lower guide edge 341 extend outwardly from the first body component 32 and the second body component 34, respectively. A guide groove 50 is formed between the upper guide edge 321 and the lower guide edge 341 for guiding the saw blade to the third cutting position C3. The upper guide edge 321 and the lower guide edge 341 are used to form the cutting track. The saw blade cuts the femur D according to the third cutting position C3, thereby operating the distal femoral osteotomy.

In the present example, the first correcting perforation 322 and the second correcting perforation 342 are designed to confirm the correction angle at which the femoral opening is opened in the distal femoral osteotomy. For this reason, there is a regulative angle N between the first hole axis A1 of the first correcting perforation 322 and the second hole axis A2 of the second correcting perforation 342. In the distal femoral osteotomy, a gap of the osteotomy has a preoperative planning correction angle. When the femur D is opened by the first body component 32 and the second body component 34 with the correction angle that is the same as the preoperative planning, the first hole axis A1 of the first correcting perforation 322 and the second hole axis A2 of the second correcting perforation 342 can coincide. An aligning bar 20 passes through the first correcting perforation 322 and the second correcting perforation 342 to ensure the opening has achieved the correction angle. Firstly, the size of the aforementioned regulative angle N is based on the correction angle that the femur D needs to open in the distal femoral osteotomy. Secondly, the size of the regulative angle N is determined according to the desired correction angle and it is made. The aligning bar 20 can be inserted between the first correcting perforation 322 and the second correcting perforation 342 only when the femur D is opened to the angle of preoperative planning by the first body component 32 and the second body component 34.

Please refer to FIG. 8A, FIG 8B, FIG. 9A and FIG. 9B. Hereby explain how to use the present example to perform the distal femoral osteotomy. In FIG. 8A and FIG. 8B, the surgical device for osteotomy 3 attached to the femoral position is located in the left thigh of human body and close to lateral distal end of the knee. However, the scope of application is not limited thereto, it can also be applied to other positions of other bones. In other words, the picture shows the positive side of FIG. 8A and FIG. 8B is pointing to the human body. The left side of FIG. 8A and FIG. 8B points to the front of the human body.

The present example of the surgical device for osteotomy 3 is based on the femoral image information provided by patients with degenerative joint disease before surgery. The model of femur D was established using computer software. The position of the third cutting position C3 is determined according to the model of femur D. The overall structure of the surgical device for osteotomy 3 is custom-made. Wherein the surgical device for osteotomy 3 is designed to be able to fit well with the surface of the patient's femur D, and then the three-dimensional printing technique is used to construct an integrally formed or combined solid instrument.

Please refer to FIG. 9A, the figure shows the completed cutting track E on the third cutting position C3. When the surgical device for osteotomy 3 is arranged on the surface of patient's femur, the surgeon inserts the saw blade and starts cutting according to the third cutting position C3 guided by the upper guide edge 321 and the lower guide edge 341 of the surgical device for osteotomy 3. The surgeon can use the upper guide edges 321 and the lower guide edges 341 as a reference for the calculation of the sawing depth. In another way, make a mark on the saw blade, surgeons can use the naked eye to confirm whether the sawing depth of the saw blade has reached the preset depth.

The saw blade keeps cutting along the upper guide edge 321 and the lower guide edge 341. Then, it cut off part of the femur D. The third cutting position C3 of the femur D is opened to the preoperative planning correction angle in the case where the surgical device for osteotomy 3 is fixed to the femur D. The aligning bar 20 is then inserted through the first correcting perforation 322 and the second correcting perforation 342. After confirming the correction angle of the osteotomy of the femur D, the gap can be fixed with the implant.

In summary, the present invention provides a surgical device for osteotomy 2. In one embodiment, it is custom-made to the skeletal image information collected from patients with degenerative joint disease before surgery. The present invention can avoid ligament injury during surgery. It can also be created to resist the rotation of the bones due to the movement. The present invention is designed according to a preoperative planning so that the surgical procedure can be simplified.

Compared with the conventional technique, the surgical device for osteotomy 1, 2, 3 is manufactured by three-dimensional printing according to the patient's skeletal image data, and the optimal cutting position is evaluated preoperatively. The device can fit well with the patient's bones. The surgeon can perform the first cutting position C1 or the third cutting position C3 under the guide groove 50 specified by the device. The guide groove 50 allows the surgeon to perform the osteotomy accurately. It also provides a reference for calculating the cutting angle and depth. The side guide edge 121, 221 provides the reference for the second cutting position C2. Not only improving the surgical accuracy and efficiency, but the present invention also standardizes the whole surgical procedure for surgeons.

The present invention is intended to cover various modifications and similar arrangements included within the scope of the appended claims.

## Claims

1. A surgery device for osteotomy (2,) comprising:
a first body component (22) having an upper guide edge (222) for forming a cutting track, wherein said upper guide edge (222) has a first correcting perforation (223);
a second body component (24) having a lower guide edge (241) disposed below said upper guide edge (222), a guide groove (50) being formed between said upper guide edge (222) and said lower guide edge (241) for guiding a saw blade to perform a cutting procedure, wherein said lower guide edge (241) has a second correcting perforation (242);
a hinge pin guide component (28) disposed on the surface of said second body component (24), wherein said hinge pin guide component (28) comprises a third correcting perforation (281) extending to the rear of said guide groove (50), said third correcting perforation (281) fits with a preoperative hinge axis (30) for osteotomy as a hinge when a bone is opened; wherein a corrective angle (L, N) between a first hole axis (A1) of said first correcting perforation (223) and a second hole axis (A2) of said second correcting perforation (242) is formed, **characterized in that** said surgery device for osteotomy (2) is configured such that when an open angle of a gap of said osteotomy is the same as that of a preoperative planning correction angle (M), said first hole axis (A1) of said first correcting perforation (223) and said second hole axis (A2) of said second correcting perforation (242) will coincide, so that an alignment bar (20) can be inserted between said first correcting perforation (223) and said second correcting perforation (242) only when a tibia is opened to said preoperative planning correction angle (M) by said first body component (22) and said second body component (24).

2. The surgical device for osteotomy (2) of claim 1, wherein said first body component (22) further comprises a side guide edge (221) for forming a cutting track.

3. The surgical device for osteotomy (2) of claim 1, wherein said first correcting perforation (223) is connected to said first body component (22) by a first bar (224); wherein said second correcting perforation (242) is connected to said second body component (24) by a second bar (243).

4. The surgical device for osteotomy (2) of claim 1, the surfaces of said first body component (22) and said second body component (24) having a plurality of fixing slots (26), said surgical device for osteotomy (2) being fixed on the surface of said bone by inserting a plurality of fixing pins (40) into said plurality of fixing slots (26).

## Patentansprüche

1. Chirurgische Vorrichtung für Osteotomie (2), umfassend:
eine erste Körperkomponente (22), die eine obere Führungskante (222) zum Bilden einer Schneidspur aufweist, wobei die obere Führungskante (222) eine erste Korrekturperforation (223) aufweist;
eine zweite Körperkomponente (24), die eine untere Führungskante (241) aufweist, die unterhalb der oberen Führungskante (222) angeordnet ist, wobei zwischen der oberen Führungskante (222) und der unteren Führungskante (241) eine Führungsnut (50) zum Führen eines Sägeblatts zum Ausführen eines Schneidvorgangs gebildet ist, wobei die untere Führungskante (241) eine zweite Korrekturperforation (242) aufweist;
eine Scharnierstift-Führungskomponente (28), die an der Oberfläche der zweiten Körperkomponente (24) angeordnet ist, wobei die Scharnierstift-Führungskomponente (28) eine dritte Korrekturperforation (281) aufweist, die sich zu der Rückseite der Führungsnut (50) erstreckt, die dritte Korrekturperforation (281) zu einer präoperativen Scharnierachse (30) für Osteotomie als ein Scharnier passt, wenn ein Knochen geöffnet wird;
wobei ein Korrekturwinkel (L, N) zwischen einer ersten Lochachse (A1) der ersten Korrekturperforation (223) und einer zweiten Lochachse (A2) der zweiten Korrekturperforation (242) gebildet ist,
**dadurch gekennzeichnet, dass** die chirurgische Vorrichtung für die Osteotomie (2) konfiguriert ist, sodass, wenn ein Öffnungswinkel eines Spalts der Osteotomie gleich ist wie der eines präoperativen Planungskorrekturwinkels (M), die erste Lochachse (A1) der ersten Korrekturperforation (223) und die zweite Lochachse (A2) der zweiten Korrekturperforation (242) übereinstimmen, sodass eine Ausrichtungsstange (20) zwischen der ersten Korrekturperforation (223) und der zweiten Korrekturperforation (242) nur dann eingeführt werden kann, wenn eine Tibia durch die erste Körperkomponente (22) und die zweite Körperkomponente (24) zu dem präoperativen Planungskorrekturwinkel (M) geöffnet ist.

2. Chirurgische Vorrichtung für Osteotomie (2) nach Anspruch 1, wobei die erste Körperkomponente (22) ferner eine seitliche Führungskante (221) zum Bilden einer Schneidspur umfasst.

3. Chirurgische Vorrichtung für Osteotomie (2) nach Anspruch 1, wobei die erste Korrekturperforation (223) durch eine erste Stange (224) mit der ersten Körperkomponente (22) verbunden ist;
wobei die zweite Korrekturperforation (242) durch eine zweite Stange (243) mit der zweiten Körperkomponente (24) verbunden ist.

4. Chirurgische Vorrichtung für Osteotomie (2) nach Anspruch 1, wobei die Oberflächen der ersten Körperkomponente (22) und der zweiten Körperkomponente (24) eine Vielzahl von Befestigungsschlitzen (26) aufweist, wobei die chirurgische Vorrichtung für Osteotomie (2) an der Oberfläche des Knochens durch Einführen einer Vielzahl von Befestigungsstiften (40) in die Vielzahl von Befestigungsschlitzen (26) befestigt wird.

## Revendications

1. Dispositif de chirurgie destiné à l'ostéotomie (2) comprenant :
un premier élément de corps (22) présentant un bord de guidage supérieur (222) destiné à former une piste de coupe, dans lequel ledit bord de guidage supérieur (222) présente une première perforation de correction (223)
un second élément de corps (24) présentant un bord de guidage inférieur (241) disposé sous ledit bord de guidage supérieur (222) une rainure de guidage (50) étant formée entre ledit bord de guidage supérieur (22) et ledit bord de guidage inférieur (241) destiné à guider une lame de scie afin d'effectuer une procédure de coupe, dans lequel ledit bord de guidage inférieur (241) présente une deuxième perforation de correction (242) ;
un élément de guidage d'axe d'articulation (28) disposé sur la surface dudit second élément de corps (24), dans lequel ledit élément de guidage d'axe d'articulation (28) comprend une troisième perforation de correction (281) s'étendant à l'arrière de ladite rainure de guidage (50), ladite troisième perforation de correction (281) s'adapte à un axe d'articulation préopératoire (30) destiné à l'ostéotomie en tant qu'une articulation lorsqu'un os est ouvert ;
dans lequel un angle de correction (L, N) entre un premier axe de trou (A1) de ladite première perforation de correction (223) et un second axe de trou (A2) de ladite deuxième perforation de correction (242) est formé ;
**caractérisé en ce que** ledit dispositif de chirurgie destiné à l'ostéotomie (2) est conçu de sorte que, lorsqu'un angle ouvert d'un espace de ladite ostéotomie est le même que celui d'un angle de correction préopératoire (M), ledit premier axe de trou (A1) de ladite première perforation de correction (223) et ledit second axe de trou (A2) de ladite deuxième perforation de correction (242) coïncident, de sorte qu'une barre d'alignement (20) peut être insérée entre ladite première perforation de correction (223) et ladite deuxième perforation de correction (242) uniquement lorsqu'un tibia est ouvert audit angle de correction de planification préopératoire (M) par ledit premier élément de corps (22) et ledit second élément de corps (24).

2. Dispositif chirurgical destiné à l'ostéotomie selon la revendication 1, dans lequel ledit premier élément de corps (22) comprend en outre un bord de guidage latéral (221) destiné à former une piste de coupe.

3. Dispositif chirurgical destiné à l'ostéotomie (2) selon la revendication 1, dans lequel ladite première perforation de correction (223) est reliée audit premier élément de corps (22) par une première barre (224) ;
dans lequel ladite deuxième perforation de correction (242) est reliée audit second élément de corps (24) par une seconde barre (243).

4. Dispositif chirurgical destiné à l'ostéotomie (2) selon la revendication 1, les surfaces dudit premier élément de corps (22) et dudit second élément de corps (24) présentant une pluralité de fentes de fixation (26), ledit dispositif chirurgical destiné à l'ostéotomie (2) étant fixé sur la surface dudit os en insérant une pluralité de broches de fixation (40) dans ladite pluralité de fentes de fixation (26).
